(19) Europäisches Patentamt
European Patent Office
Office européen des brevets

(11) Veröffentlichungsnummer: **0 493 782 A2**

(12) # EUROPÄISCHE PATENTANMELDUNG

(21) Anmeldenummer: **91122153.9**

(51) Int. Cl.5: **C07D 211/90**, A61K 31/445

(22) Anmeldetag: **23.12.91**

(30) Priorität: **04.01.91 DE 4100125**

(43) Veröffentlichungstag der Anmeldung:
**08.07.92 Patentblatt 92/28**

(84) Benannte Vertragsstaaten:
**AT BE CH DE DK ES FR GB GR IT LI LU NL SE**

(71) Anmelder: **BAYER AG**
**W-5090 Leverkusen 1 Bayerwerk(DE)**

(72) Erfinder: **Stoltefuss, Jürgen, Dipl.-Ing.**
**Parkstrasse 20**
**W-5657 Haan 2(DE)**
Erfinder: **Hirth-Dietrich, Claudia, Dr.**
**Claudiusweg 9**
**W-5600 Wuppertal(DE)**
Erfinder: **Stasch, Johannes-Peter, Dr.**
**Schneewittchenweg 37**
**W-5600 Wuppertal(DE)**
Erfinder: **Neuser, Dieter, Dr.**
**29312 Channel View Drive**
**Elkhart, Indiana 46516(US)**

(54) **Neue Dihydropyridinamide, Verfahren zu ihrer Herstellung und ihre Verwendung in Arzneimitteln.**

(57) Die vorliegende Erfindung betrifft neue Dihydropyridinamide der allgemeinen Formel I

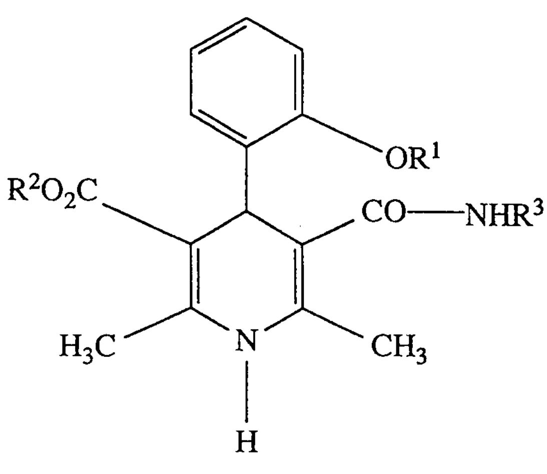

in welcher $R^1$, $R^2$ und $R^3$ die in der Beschreibung angegebene Bedeutung haben, Verfahren zu ihrer Herstellung und ihre Verwendung in Arzneimitteln, insbesondere ihre Verwendung als kreislaufbeeinflussende Arzneimittel.

EP 0 493 782 A2

Die vorliegende Erfindung betrifft neue Dihydropyridinamide, Verfahren zu ihrer Herstellung und ihre Verwendung in Arzneimittel, insbesondere ihre Verwendung als kreislaufbeeinflussende Arzneimittel.

Es ist bereits bekannt, daß 3-Aminocarbonyl-1,4-dihydropyridin-5-carbonsäureesterderivate eine antiarrhythmische und blutdrucksenkende Wirkung besitzten [vgl. J 01075-467-A und EP 288 758 A2]. Kreislaufwirksame Dihydropyridinamide sind beschrieben in DOS 2 228 377 und auch in EP-A-220 653.

Außerdem ist bekannt, daß das Polypeptidhormon ANP (atriales, natriuretisches Peptid) Diurese, Natriurese und Vaskorelavation induzieren kann [vgl. Nature 338, 78-83, 1989; Biochem. J. 232, 313-321 (1985)]. Diese Wirkung wird durch zyklisches Guanosin-Monophosphat (cGMP) vermittelt, dessen renale Ausscheidung einen spezifischen Marker für die Aktivierung des ANP-Systems darstellt. [vgl. Europ. J. Pharmacol. 129, 165-168, 1986 und Am. J. Physiol. 255 F1220-F1224, 1988].

Die vorliegende Erfindung betrifft nun neue Dihydropyridinamide der allgemeinen Formal (I)

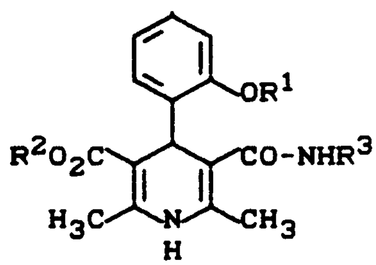

in welcher

$R^1$
- für geradkettiges oder verzweigtes Alkyl mit bis zu 6 Kohlenstoffatomen steht, das gegebenenfalls durch Phenoxy oder Cyclohexyl substituiert ist, oder
- für Wasserstoff oder Trifluormethyl steht,

$R^2$
- für geradkettiges oder verzweigtes Alkyl mit bis zu 4 Kohlenstoffatomen steht, das gegebenenfalls durch Cyano, Hydroxy oder Acetoxy substituiert ist,

$R^3$
- für geradkettiges oder verzweigtes Alkyl mit bis zu 5 Kohlenstoffatomen steht, oder
- für Cycloalkyl mit 3 bis 5 Kohlenstoffatomen steht.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

$R^1$
- für geradkettiges oder verzweigtes Alkyl mit bis zu 4 Kohlenstoffatomen steht, das gegebenenfalls durch Phenoxy oder Cyclohexyl substituiert ist, oder
- für Wasserstoff oder Trifluormethyl steht,

$R^2$
- für geradkettiges oder verzweigtes Alkyl mit bis zu 3 Kohlenstoffatomen steht, das gegebenenfalls durch Cyano, Hydroxy oder Acetoxy substituiert ist,

$R^3$
- für geradkettiges oder verzweigtes Alkyl mit bis zu 4 Kohlenstoffatomen steht, oder
- für Cyclopropyl oder Cyclopentyl steht.

Besonders bevorzugt sind Verbindungen der allgemeinen Formel (I),
in welcher

$R^1$
- für geradkettiges oder verzweigtes Alkyl mit bis zu 4 Kohlenstoffatomen steht, das gegebenenfalls durch Cyclohexyl oder Phenoxy substituiert ist, oder
- für Wasserstoff oder Trifluormethyl steht,

$R^2$
- für Methyl, Ethyl oder Propyl steht,

$R^3$
- für Methyl, Ethyl, Propyl, Isopropyl, Cyclopropyl oder Cyclopentyl steht.

Außerdem wurden Verfahren zur Herstellung der erfindungsgemäßen Verbindungen der allgemeinen Formel (I) gefunden, dadurch gekennzeichnet, daß man

[A] Aldehyde der allgemeinen Formel (II)

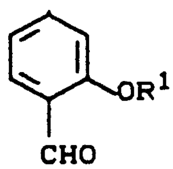

(II)

in welcher
$R^1$ die oben angegebene Bedeutung hat,
entweder mit $\beta$-Ketocarbonsäureestern der allgemeinen Formel (III)

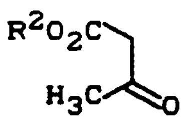

(III)

in welcher
$R^2$ die oben angegebene Bedeutung hat,
und $\beta$-Ketocarbonsäureamiden der allgemeinen Formel (IV)

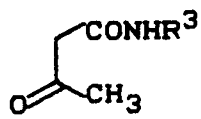

(IV)

in welcher
$R^3$ die oben angegebene Bedeutung hat,
und Ammoniak umsetzt, oder
die Verbindungen der allgemeinen Formeln (II) und (III) direkt mit Enaminocarbonsäureamiden der allgemeinen Formel (V)

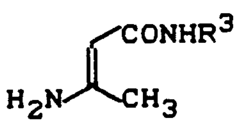

(V)

in welcher
$R^3$ die oben angegebene Bedeutung hat,
gegebenenfalls in Anwesenheit eines inerten Lösemittels umsetzt,
oder
zunächst als Zwischenprodukte Verbindungen der allgemeinen Formel (VI)

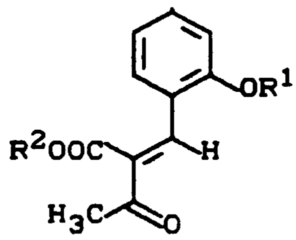

(VI)

in welcher
$R^1$ und $R^2$ die oben angegebene Bedeutung haben,
herstellt und diese dann mit Verbindungen der allgemeinen Formel (V) umsetzt
oder
[B] zunächst Yliden-$\beta$-ketocarbonsäureamide der allgemeinen Formel (VII) herstellt,

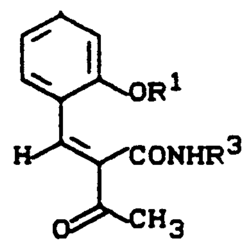

(VII)

in welcher
$R^1$ und $R^3$ die oben angegebene Bedeutung haben,
und diese mit Verbindungen der allgemeinen Formel (VIII),

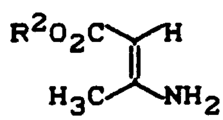

(VIII)

in welcher
$R^2$ die oben angegebene Bedeutung hat,
in inerten Lösemitteln umsetzt,
oder
[C] Dihydropyridinmonocarbonsäuren der allgemeinen Formel (IX)

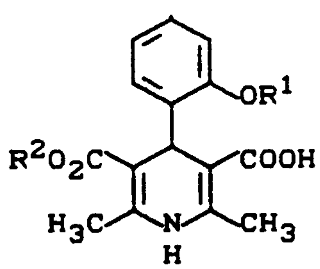

(IX)

in welcher
$R^1$ und $R^2$ die oben angegebene Bedeutung haben,
gegebenenfalls über ein reaktives Säurederivat mit Aminen der allgemeinen Formel (X)

$H_2N\text{-}R^3$ (X)

in welcher
$R^3$ die oben angegebene Bedeutung hat,
gegebenenfalls in Anwesenheit eines inerten organischen Lösemittels umsetzt.

Die erfindungsgemäßen Verfahren können durch folgendes Formelschema beispielhaft erläutert werden:

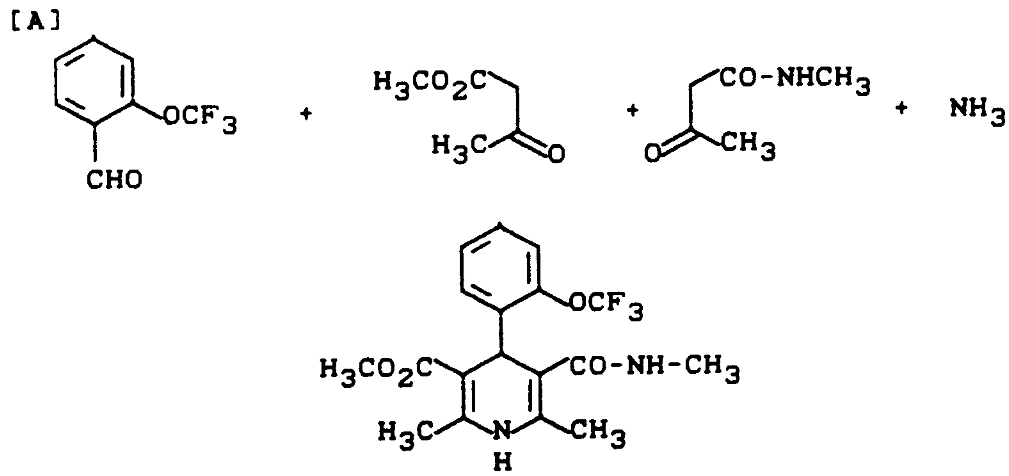

[B]

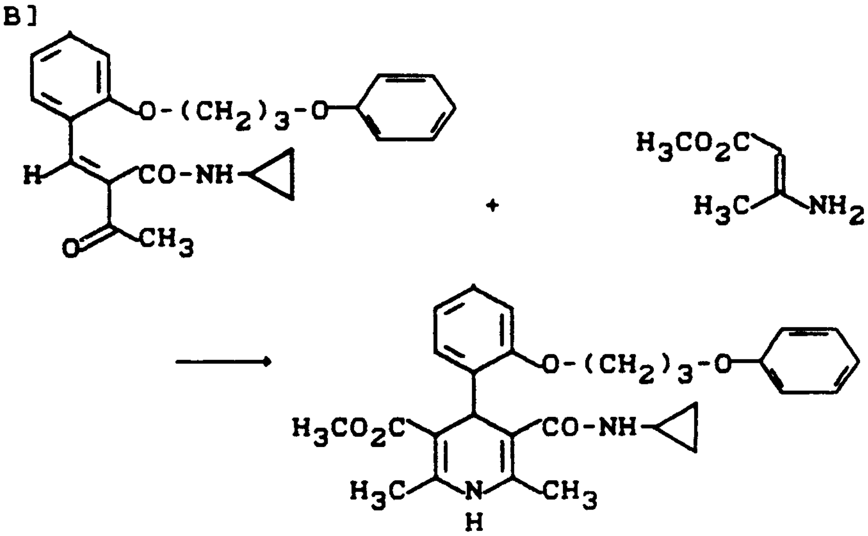

[C]

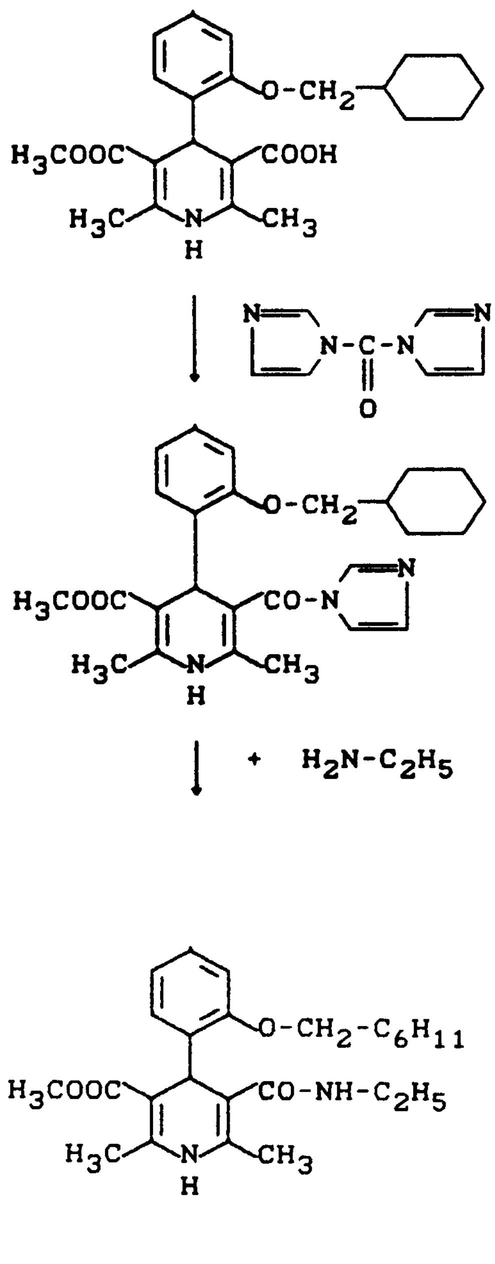

Verfahrensvarianten A und B

Als Lösemittel kommen Wasser oder alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A und B ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die als Ausgangsstoffe eingesetzten Aldehyde der allgemeinen Formel (II) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten $\beta$-Ketocarbonsäureester der allgemeinen Formel (III) sind bekannt oder können nach bekannten Methoden hergestellt werden [D. Borrmann in Houben Weyl's "Methoden der organischen Chemie" Bd. VII/4, 230 (1968); Y. Oikawa, K. Sugano, O. Yonemitsu, J. Org. Chem. 43, 2087 (1978)].

Die als Ausgangsstoffe eingesetzten $\beta$-Ketocarbonsäureamide der allgemeinen Formel (IV) sind bekannt oder können nach bekannten Methoden hergestellt werden [DOS 11 42 859].

Die als Ausgangsstoffe eingesetzten Enaminocarbonsäureamide der allgemeinen Formel (V) sind bekannt oder können nach bekannten Methoden hergestellt werden [DOS 2 228 377].

Die als Ausgangsstoffe eingesetzten Enaminocarbonsäureester der allgemeinen Formel (VIII) sind bekannt oder können nach bekannten Methoden hergestellt werden [F.A. Glickman, A.G. Cope, J. Am. Chem. Soc, 67, 1017 (1945)].

Die als Ausgangsstoffe eingesetzten Yliden-$\beta$-ketocarbonsäureamide der allgemeinen Formel (VII) und die als Ausgangsstoffe eingesetzten Yliden-$\beta$-ketocarbonsäureester der allgemeinen Formel (VI) sind bekannt oder können nach bekannten Methoden hergestellt werden [G. Jones "The Knoevenagel Condensation" in Organic Reactions Bd. XV, 204 (1967)].

Die Durchführung der erfindungsgemäßen Verfahrensvariante C lehnt sich an die literaturbekannte Methodik zur Überführung von Carbonsäuren in Carbonsäureamide an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt werden, oder die in situ direkt zu den erfindungsgemäßen Verbindungen amidiert werden. Als aktivierende Reagenzien seien neben den anorganischen Halogeniden wie Thionylchlorid, Phosphortrichlorid oder Phosphorpentachlorid, oder dem Carbonyldiimidazol, Carbodiimide wie Cyclohexylcarbodiimid oder 1-Cyclohexyl-3-[2-(N-methyl-morpholino)-ethyl]-carbodiimid-p-toluolsulfonat oder N-Hydroxy-phthalimid oder N-Hydroxy-benztriazol in Gegenwart von Dicyclohexylcarbodiimid beispielhaft erwähnt. Naturgemäß lassen sich die Dihydropyridinmonocarbonsäuren auch in Form ihrer Salze einsetzen. [Die Methodik der Amidierung wird beispielsweise beschrieben von: Fieser & Fieser, Reagents for Organic Synthesis, John Wiley & Sons Inc. (1967), Seite 231 - 236; J.C. Shihan and G.P. Hess, J. Am. Chem. Soc. 77, 1067 (1955); U. Goodman, G.W. Kenner, Adv. in Protein Chem. 12, 488 (1957); W.A. Bonner, P.I. McNamee, J. Org. Chem. 26, 254 (1961); H.A. Staab, Angew. Chemie Int. Ed. 1, 351 (1962); Fieser & Fieser, Reagents for Organic Synthesis, John Wiley & Sons Inc, 1967, 116, 114; H.C. Beyerman, U.O. van der Brink, Re. Trav. 80, 1372 (1961); C.A. Buehler, D.E. Pearson, John Wiley & Sons, Volume I (1970), Seite 895 ff, Volume II, (1977)].

Als Lösemittel für Verfahrensvariante C kommen neben Wasser alle inerten organischen Lösemittel in Frage, die sich unter den Reaktionsbedingungen nicht verändern. Hierzu gehören vorzugsweise Ether wie Diethylether, Dioxan, Tetrahydrofuran, Glykoldimethylether, oder halogenierte Kohlenwasserstoffe wie Dichlormethan, Trichlormethan oder Tetrachlormethan, oder Amide wie Dimethylformamid, Dimethylacetamid oder Hexamethylphosphorsäuretriamid, oder Kohlenwasserstoffe wie Benzol, Toluol oder Xylol, oder Acetonitril, Nitromethan, Pyridin, Dimethylsulfoxid oder Essigester. Ebenso können Gemische der genannten Lösemittel verwendet werden. Isoliert man die aktivierten Zwischenstufen der Dihydropyridinmonocarbonsäuren, so können auch die Amine der Formel (X) alleine als Verdünnungsmittel verwendet werden.

Die Reaktionstemperaturen können in einem breiten Bereich variiert werden. Im allgemeinen arbeitet man in einem Bereich von -70 °C bis +140 °C, bevorzugt von -20 °C bis +100 °C.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvariante C ist das Verhältnis der an der Reaktion beteiligten Stoffe beliebig. Im allgemeinen arbeitet man jedoch mit molaren Mengen der Reaktanden. Es hat sich jedoch als günstig erwiesen, das Amin in einem 5- bis 10-fach molaren Überschuß einzusetzen. Besonders zweckmäßig wird das Amin in einem großen Überschuß direkt als Lösemittel eingesetzt.

Die als Ausgangsstoffe eingesetzten Dihydropyridinmonocarbonsäuren der allgemeinen Formel (IX) sind bekannt oder können nach bekannten Methoden hergestellt werden [DOS 2 847 236; 3 206 671; 2 962 241].

Die als Ausgangsstoffe eingesetzten Amine der allgemeinen Formel (X) sind bekannt oder können nach bekannten Methoden hergestellt werden [Houben Weyl's "Methoden der organischen Chemie" Bd XI/1; Paulsen, Angewandte Chemie 78, 501 - 566 (1966)].

Die ausgewählten erfindungsgemäßen Verbindungen zeigen ein nicht vorhersehbares, wertvolles pharmakologisches Wirkspektrum. Im Vergleich zu strukturähnlichen, bekannten Dihydropyridinen wie z.B. in DOS 2 228 377 beschriebenen Vertretern, zeigen die erfindungsgemäßen Verbindungen sowohl quantitativ als auch qualitativ ein anderes Wirkungsspektrum. Neben ihrer calciumantagonistischen Wirkung steigern sie die ANP-abhängige Natriurese bei Stimulation des ANP-Systems durch Volumenbelastung. Diese zusätzliche Wirkkomponente wurde in einem Modell der akuten Hypervolämie an der Ratte nachgewiesen: Die Testsubstanzen wurden oral in Tylose-Suspension verabreicht. Durch Injektion von 15 ml/kg physiologischer Kochsalzlösung in die Schwanzvene wurde eine akute Volumenbelastung ausgelöst und anschließend der Urin über eine Stunde gesammelt. Unerwarteterweise verstärken die erfindungsgemäßen Verbindungen die Natriumausscheidung. Gleichzeitig wird die Ausscheidung von cGMP, das einen spezifischen Marker für die Aktivierung des ANP-Systems darstellt, erhöht (z.B. um 100 % bei Beispiel 19).

Tabelle I:

| Beispiel-Nr. | Dosis mg/kg p.o. | Steigerung der $Na^+$-Ausscheidung % |
|---|---|---|
| 2 | 10 | 80 |
| 11 | 10 | 95 |
| 19 | 10 | 100 |

Die erfindungsgemäßen Verbindungen können deshalb in Arzneimitteln zur Behandlung des pathologisch veränderten Blutdrucks und der Herzinsuffizienz, sowie als Koronartherapeutika eingesetzt werden.

Darüber hinaus können sie zur Behandlung von Herzrhythmusstörungen, Niereninsuffizienz, Leberzirrhose, Aszites, Lungenödem, Hirnödem, Schwangerschaftsödem, Glaukom oder Diabetes mellitus eingesetzt werden,

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher weise, vorzugsweise oral oder parenteral, insbesondere perlingual oder intravenös.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

1,4-Dihydro-2,6-dimethyl-4-[2-(2-phenoxy-ethoxy)]phenyl-3-cyclopropyl-carbamoyl-pyridin-5-carbonsäuremethylester

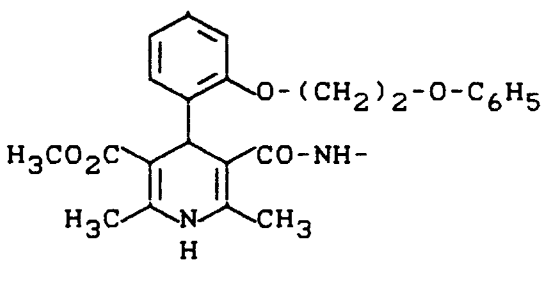

4,8 g (14,1 mmol) 2-(2-Phenoxy-ethoxy)-benzylidenacetessigsäuremethylester werden in 40 ml Isopropanol mit 1,97 g (14,1 mmol) 3-Amino-carbonsäurecyclopropylamid über Nacht gekocht. Es wird eingeengt und über eine Kieselgelsäule mit Toluol/Essigester-Gemischen gereinigt. Die sauberen Fraktionen werden gesammelt und eingeengt. Das Produkt kristallisiert aus Isopropanol. Man erhält 3,0 g farblose Kristalle vom Schmelzpunkt 151 ° C.

Die in Tabelle 1 aufgeführten Beispiele wurden in Analogie zur Vorschrift des Beispiels 1 hergestellt:

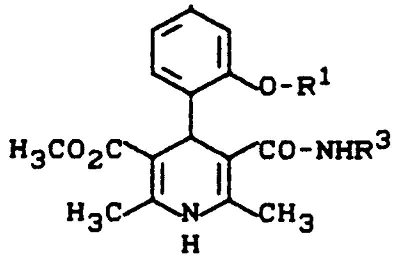

| Bsp.-Nr. | $R^1$ | $R^3$ | F (°C) |
|---|---|---|---|
| 2 | $-(CH_2)_3-O-C_6H_5$ | 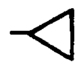 | 178 |
| 3 | $-(CH_2)_2-O-C_6H_5$ | $-C_2H_5$ | 135 |
| 4 | $-CH_2-$ 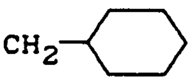 | 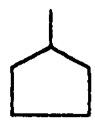 | 165 |
| 5 | H | 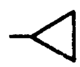 | 184 |
| 6 | $CF_3$ | 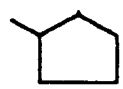 | 257 |
| 7 | $CF_3$ | $-C_3H_7$ | 251 |
| 8 | $CF_3$ | $-CH_3$ | 236 |
| 9 | $CH_3$ | $-C_2H_5$ | 173-175 |
| 10 | $CH_3$ | $-C_3H_7$ | 174 |

| Bsp.-Nr. | $R^1$ | $R^3$ | F (°C) |
|---|---|---|---|
| 11 | $CH_3$ | 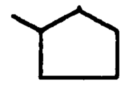 | 185-187 |
| 12 | $-CH_2-$ 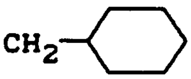 | 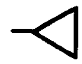 | 169 |
| 13 | $-CH_2-$ 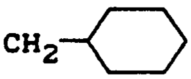 | $-C_3H_7$ | 145 |
| 14 | $-CH_2-$ 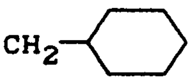 | $-C_2H_5$ | 150 |
| 15 | $-CH_3$ | $-CH_3$ | 186 |
| 16 | $-CH_2-$ 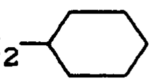 | $-CH_3$ | 110 |
| 17 | $-CH_3$ | 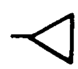 | 192 |
| 18 | $-CF_3$ | $-C_2H_5$ | 240-242 |
| 19 | $-CF_3$ | 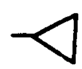 | 243-245 |

## Patentansprüche

1. Dihydropyridinamide der allgemeinen Formel (I)

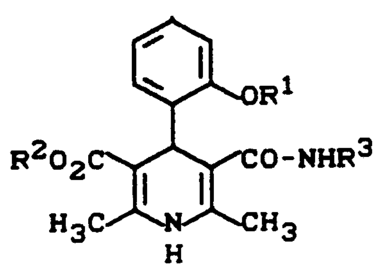

in welcher

$R^1$

- für geradkettiges oder verzweigtes Alkyl mit bis zu 6 Kohlenstoffatomen steht, das gegebenenfalls durch Phenoxy oder Cyclohexyl substituiert ist, oder

- für Wasserstoff oder Trifluormethyl steht,

$R^2$

- für geradkettiges oder verzweigtes Alkyl mit bis zu 4 Kohlenstoffatomen steht, das gegebenenfalls durch Cyano, Hydroxy oder Acetoxy substituiert ist,

$R^3$

- für geradkettiges oder verzweigtes Alkyl mit bis zu 5 Kohlenstoffatomen steht, oder
- für Cycloalkyl mit 3 bis 5 Kohlenstoffatomen steht,

in Racemform und in Form ihrer optischen Antipoden.

**2.** Verbindungen der allgemeinen Formel (I) gemäß Anspruch 1
in welcher

$R^1$

- für geradkettiges oder verzweigtes Alkyl mit bis zu 4 Kohlenstoffatomen steht, das gegebenenfalls durch Phenoxy oder Cyclohexyl substituiert ist, oder
- für Wasserstoff oder Trifluormethyl steht,

$R^2$

- für geradkettiges oder verzweigtes Alkyl mit bis zu 3 Kohlenstoffatomen steht, das gegebenenfalls durch Cyano, Hydroxy oder Acetoxy substituiert ist,

$R^3$

- für geradkettiges oder verzweigtes Alkyl mit bis zu 4 Kohlenstoffatomen steht, oder
- für Cyclopropyl oder Cyclopentyl steht.

**3.** Verbindungen der allgemeinen Formel (I) gemäß Anspruch 1,
in welcher

$R^1$

- für geradkettiges oder verzweigtes Alkyl mit bis zu 4 Kohlenstoffatomen steht, das gegebenenfalls durch Cyclohexyl oder Phenoxy substituiert ist, oder
- für Wasserstoff oder Trifluormethyl steht,

$R^2$

- für Methyl, Ethyl oder Propyl steht,

$R^3$

- für Methyl, Ethyl, Propyl, Isopropyl, Cyclopropyl oder Cyclopentyl steht.

**4.** Verfahren zur Herstellung von Verbindungen der allgemeinen Formel (I)

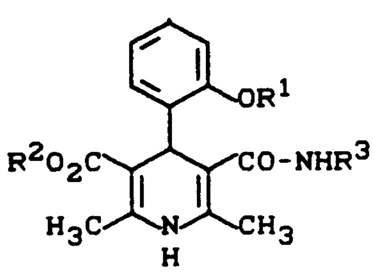

in welcher

$R^1$

- für geradkettiges oder verzweigtes Alkyl mit bis zu 6 Kohlenstoffatomen steht, das gegebenenfalls durch Phenoxy oder Cyclohexyl substituiert ist, oder
- für Wasserstoff oder Trifluormethyl steht,

$R^2$

- für geradkettiges oder verzweigtes Alkyl mit bis zu 4 Kohlenstoffatomen steht, das gegebenenfalls durch Cyano, Hydroxy oder Acetoxy substituiert ist,

$R^3$

- für geradkettiges oder verzweigtes Alkyl mit bis zu 5 Kohlenstoffatomen steht, oder
- für Cycloalkyl mit 3 bis 5 Kohlenstoffatomen steht,

dadurch gekennzeichnet, daß man

[A] Aldehyde der allgemeinen Formel (II)

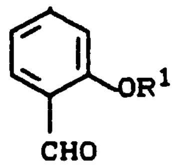

(II)

in welcher
$R^1$ die oben angegebene Bedeutung hat,
entweder mit β-Ketocarbonsäureestern der allgemeinen Formel (III)

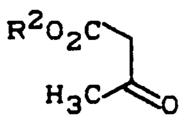

(III)

in welcher
$R^2$ die oben angegebene Bedeutung hat,
und β-Ketocarbonsäureamiden der allgemeinen Formel (IV)

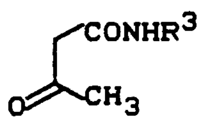

(IV)

in welcher
$R^3$ die oben angegebene Bedeutung hat,
und Ammoniak umsetzt, oder
die Verbindungen der allgemeinen Formeln (II) und (III) direkt mit Enaminocarbonsäureamiden der allgemeinen Formel (V)

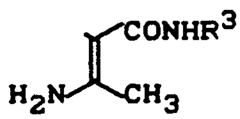

(V)

in welcher
$R^3$ die oben angegebene Bedeutung hat,
gegebenenfalls in Anwesenheit eines inerten Lösemittels umsetzt,
oder
zunächst als Zwischenprodukte Verbindungen der allgemeinen Formel (VI)

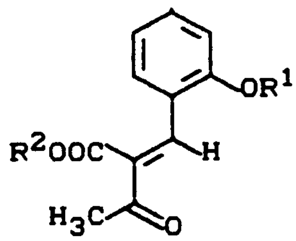

(VI)

in welcher
$R^1$ und $R^2$ die oben angegebene Bedeutung haben,
herstellt und diese dann mit Verbindungen der allgemeinen Formel (V) umsetzt
oder

[B] zunächst Yliden-β-ketocarbonsäureamide der allgemeinen Formel (VII) herstellt,

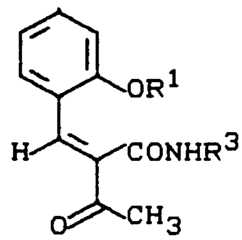

(VII)

in welcher
$R^1$ und $R^3$ die oben angegebene Bedeutung haben,
und diese mit Verbindungen der allgemeinen Formel (VIII),

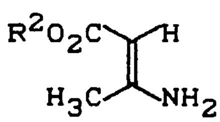

(VIII)

in welcher
$R^2$ die oben angegebene Bedeutung hat,
in inerten Lösemitteln umsetzt,
oder

[C] Dihydropyridinmonocarbonsäuren der allgemeinen Formel (IX)

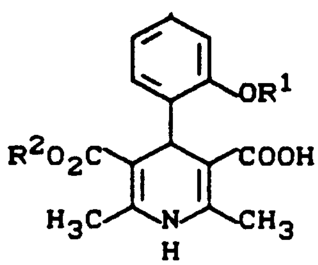

(IX)

in welcher
$R^1$ und $R^2$ die oben angegebene Bedeutung haben,

gegebenenfalls über ein reaktives Säurederivat mit Aminen der allgemeinen Formel (X)

$H_2N\text{-}R^3$ (X)

in welcher
$R^3$ die oben angegebene Bedeutung hat,
gegebenenfalls in Anwesenheit eines inerten organischen Lösemittels umsetzt.

**5.** Verfahren gemäß Anspruch 4, dadurch gekennzeichnet, daß man für die Verfahrensvarianten [A] und [B] die Reaktion in einem Temperaturbereich von +10 bis 150 °C durchführt und die Verfahrensvariante [C] in einem Temperaturbereich von -70 bis 100 °C durchführt.

**6.** Arzneimittel, enthaltend mindestens eine Verbindung der allgemeinen Formel (I), gemäß Anspruch 1.

**7.** Verfahren zur Herstellung von Arzneimitteln, dadurch gekennzeichnet, daß man Verbindungen der allgemeinen Formel (I) gemäß Anspruch 1, gegebenenfalls mit üblichen Hilfs- und Zuschlagstoffen, in eine geeignete Applikationsform überführt.

**8.** Verbindungen der allgemeinen Formel (I) gemäß Anspruch 1, zur Verwendung bei der Bekämpfung von Krankheiten.

**9.** Verwendung von Verbindungen der allgemeinen Formel (I) gemäß Anspruch 1, bei der Herstellung von Arzneimitteln zur Behandlung von Kreislauferkrankungen.

**10.** Verwendung von Verbindungen der allgemeinen Formel (I) gemäß Anspruch 1, bei der Herstellung von Arzneimitteln zur Freisetzung des atrialen natriuretischen Peptids.